# EUROPEAN PATENT SPECIFICATION

(11) **EP 2 092 293 B1**
(45) Date of publication and mention of the grant of the patent: **19.08.2015**
(21) Application number: 07812664.6
(22) Date of filing: 06.07.2007
(51) Int. Cl.: G01N 15/04, G01N 33/49

(54) **BLOOD PROCESSING APPARATUS WITH ROBUST OUTFLOW PROCESS CONTROL**
BLUTAUFARBEITUNGSVORRICHTUNG MIT ROBUSTER AUSLAUFPROZESSKONTROLLE
APPAREIL DE TRAITEMENT DU SANG AVEC COMMANDE DE PROCESSUS d'écoulement de sortie ROBUSTE

(30) Priority: 17.08.2006 US 822672 P; 12.04.2007 US 911362 P; 02.07.2007 US 772692
(43) Date of publication of application: 26.08.2009
(73) Proprietor: Terumo BCT, Inc., Lakewood, CO 80215 (US)
(72) Inventor: LINDNER, John R., Morrison, CO 80465 (US); KOLENBRANDER, Jeremy, Brighton, Colorado 80602 (US); SWEAT, William, Lakewood, CO 80228 (US)
(74) Representative: Roberts, Mark Peter
(86) International application number: PCT/US2007/072916
(87) International publication number: WO 2008/021633

(56) References cited:
- EP-A- 1 146 748
- WO-A-02/13139
- WO-A-99/08091
- WO-A-2006/071302
- JP-A- 1 216 242
- US-A- 5 414 778
- US-A- 5 980 757
- US-A- 6 026 174
- US-A- 6 078 680
- US-A1- 2002 031 255
- US-A1- 2002 058 575
- US-A1- 2002 148 787
- US-A1- 2004 153 128
- US-A1- 2005 051 466
- US-B1- 6 707 952
- PERONA AND MALIK: "scale-space and edge detection using anisotropic diffusion" IEEE TRANSACTIONS ON PATTERN ANALYSIS AND MACHINE INTELLIGENCE, vol. 12, no. 7, 1990, pages 629-639, XP002468753

## Description

### Cross Reference to Related Applications

This application claims the benefit of U.S. Provisional Application No. 60/911,362 filed April 12, 2007. This application is also a continuation-in-part of U.S. Application No. 11/772,692, filed July 3, 2007, which claims the benefit of U.S. Provisional Application No. 60/822,672 filed August 17, 2006.

### Field of the Invention

The present invention relates to an apparatus and method for separating particles or components of a fluid. The invention has particular advantages in connection with separating blood components, such as white blood cells and platelets.

### Description of the Related Art

In the medical field, it is often necessary to separate blood into components. Whole blood consists of various liquid components and particle components. The liquid portion of blood is largely made up of plasma, and the particle components include red blood cells (erythrocytes), white blood cells (leukocytes), and platelets (thrombocytes). While these constituents have similar densities, their average density relationship, in order of decreasing density, is as follows: red blood cells, white blood cells, platelets, and plasma. In addition, the particle components are related according to size, in order of decreasing size, as follows: white blood cells, red blood cells, and platelets.

Typically, donated platelets are separated or harvested from other blood components using a centrifuge. White cells or other selected components may also be harvested. The centrifuge rotates a blood separation vessel to separate components within the vessel or reservoir using centrifugal force. In use, blood enters the separation vessel while it is rotating rapidly and centrifugal force stratifies the blood components, so that particular components may be separately removed. Components are removed through ports arranged within stratified layers of blood components.

White blood cells and platelets in plasma form a medium density stratified layer or "buffy coat". Because typical centrifuge collection processes are unable to consistently and satisfactorily separate white blood cells from platelets in the buffy coat, other processes have been added to improve results. One separation process is one known as centrifugal elutriation. In one common form of elutriation, a cell batch is introduced into a flow of liquid elutriation buffer, which carries the cell batch in suspension into a funnel-shaped chamber located on a spinning centrifuge. As additional liquid buffer solution flows through the chamber, the liquid sweeps smaller sized, slower-sedimenting cells toward an elutriation boundary within the chamber, while larger, faster-sedimenting cells migrate to an area of the chamber having the greatest centrifugal force.

When the centrifugal force and force generated by the fluid flow are balanced, the fluid flow is increased to force slower-sedimenting cells from an exit port in the chamber, while faster-sedimenting cells are retained in the chamber. If fluid flow through the chamber is increased, progressively larger, faster-sedimenting cells may be removed from the chamber.

The apparatus has a fluid separation chamber having a first frustro-conical segment adjacent a fluid inlet and radially inward therefrom, a second frustro-conical segment immediately adjacent the first frustro-conical segment and radially inward therefrom, the second frustro conical segment having a taper such that particles within the second frustro-conical segment are subjected to substantially equal and opposite centripetal and fluid flow forces. The taper of the second frustro-conical segment is selected based on the expected size of particles, such that at least particles of the average size of expected particles will be subjected to substantially equal and opposite centripetal and fluid forces. The apparatus has at least one pump controlling a rate of fluid flow through the fluid separation chamber, a camera configured to observe fluid flow with respect to the fluid separation chamber, and a controller receiving signals from the camera and controlling the motor and the pump.

For these and other reasons, there is a need to improve control of particle separation and/or separation of components of a fluid.

Additional technology related to this application is disclosed in, for example, U.S. Patent No. 5,722,926, issued Mar. 3,1998; U.S. Patent No. 5,951,877, issued Sep. 14, 1999; U.S. patent 6,053,856, issued April 25, 2000; U.S. patent 6,334,842, issued January 1, 2002; U.S. patent application Ser. No. 10/905,353, filed December 29,2004; U.S. patent application Ser. No. 11/163,969, filed November 4,2005 and in particular U.S. patent application Ser. No. 10/884,877, filed July 1, 2004.

The invention is defined in the claims.

The present invention comprises a blood component separation apparatus having a rotor for centrifugally separating blood into phases such as red blood cells, white blood cells or buffy coat, or plasma. A camera monitors a separation chamber and image processing determines the location of boundaries. The apparatus controls the position of the boundaries by adjusting the speed of pumps or the rotor or both.

In the present invention, fluid flow in a blood separation chamber in a centrifugal separation device is selectively controlled by optical sensing of two regions in the separation chamber. Interface position may be controlled by optical sensing of a two-dimensional view of the interface in the separation chamber in an area adjacent an outflow port or ports. Gross adjustments, that is, relatively large changes in the location of the interface or interfaces are best controlled by this observation of the interface. Thus in transient states, such as the initial setup of flow conditions, interface position sensing can be effective. Fluid flow may also be controlled in response to the optical intensity (light or dark) of the fluid in the outflow tube. This optical intensity correlates to presence of certain blood components such as red blood cells. Fine adjustments, that is, relatively small changes in the location of the interface are best controlled by sensing the optical intensity in the outflow tube. Thus in steady state conditions, such as the extraction of a blood component through the outflow tube, outflow intensity sensing is more effective. The present invention uses both methods and selects between the methods to provide optimum process control.

In a high-speed centrifuge for separating blood components, control of the interface between blood components presents significant control problems. The present apparatus controls the interface location by measuring light intensity in at least a first flux monitoring region in the collect port whereby the general level of the interface is set by, for example, detecting the presence or absence of RBC's in the collect port, and then by monitoring the interface in the phase boundary or interface monitoring region. The location of the interface is reliably detected by a series of image processing steps, which allow the apparatus to recognize a boundary or interface despite the high speed of the centrifuge rotor, the stroboscopic light used for observation, and the limitations of data processing time caused by the need for real-time response to changes in the interface location. Monitoring the interface in the interface monitoring region allows the apparatus to control the location of the interface with stability. The image processing steps for controlling the interface may comprise the steps of "spoiling" the image, "diffusing" the image, "edge detection", "edge linking", "region-based confirmation", and "interface calculation". These image processing steps will be described herein in connection with general flow chart representations for clarity. It will be understood that one skilled in the art would implement the programs in a selected programming language, such as C++ for example, but the programs could also be implemented in machine language, in firmware, or in dedicated circuitry without departing from the teachings set forth herein. "Spoiling" the image reduces the number of pixels to be examined preferentially on orthogonal axes oriented with respect to the expected location of the interface or phase boundary. For example, if the pixels of the image are oriented in rows parallel to the interface and in columns perpendicular to the interface or interfaces, the software might sample every third pixel along rows and every tenth pixel along columns or, more preferably, every pixel along rows and every tenth pixel along columns. This reduces the number of pixels to be processed, while retaining sufficient detail in a preferred direction to detect changes in the interface location. "Diffusing" the image smoothes out small oscillations in the interface boundary, making the location of the interface more distinct. "Edge detection" computes the rate of change in pixel intensity (that is, the derivative of the pixel intensity) as a function of distance in x (parallel to rotation) and y (perpendicular to rotation or radially with respect to the centrifuge) directions. Locations where the derivatives reach maxima indicate sharp intensity changes between pixels, which may represent an interface. "Edge linking" connects adjacent maxima. A chain of such connected maxima is identified as an edge of an interface if the chain is sufficiently long. The length may be predetermined empirically. Short chains are ignored as waves or other flow phenomenon. To confirm that the boundaries have actually been detected, the software uses the potential boundaries to form regions on either sides of the boundary and determines the average intensity of pixels in each region. "Region-based confirmation" creates a pseudo image of the regions that qualify as distinct, that is, having at least a pre-determined difference in average intensity, and shades each region differently. "Final edge calculation" uses the points where the shade changes in the pseudo image, averages the y (radial) displacement of these points and recognizes this average radial location as the true interface position.

This image processing is fast enough to respond to the high speed of the centrifuge in real time, yet sufficiently robust to detect subtle changes in the location of the interface or interfaces such that rotor speed or pump speed can be changed to correct and control the interface location. Responding to changes in intensity in the flux monitoring region would not be rapid enough to maintain the quality of blood product being collected.

Collect port measuring of intensity in the flux monitoring region allows measurement of cellular material leaving through the collect port in real time. Statistical measures may be used for such parameters as the hematocrit of collected blood product, allowing for more accurate collection of a desired type of product.

There is disclosed a density centrifuge blood processing system for separating fluid components comprising a separation chamber rotating about a central rotation axis, said separation chamber having an outflow passage, at least one pump regulating fluid flow in said separation chamber, a light source in optical communication with the density centrifuge blood processing system, the light source providing an incident light beam for illuminating an observation region on said density centrifuge blood processing system and a viewing region on said outflow passage, a first detector in optical communication with said separation chamber to receive and detect said light from said observation region, a second detector in optical communication with said outflow passage, a computational apparatus distinguishing one or more phase boundaries in said observation region and further distinguishing fluid composition in said viewing region as a function of light intensity received from the viewing region, and a controller regulating speed of said at least one pump or of said separation chamber in response to signals from said computational apparatus.

There is provided a centrifuge blood processing system comprising a camera having a two-dimensional field of view, and wherein a first detector comprises a first discrete area in a field of view and a second detector comprises a second discrete area in the field of view.

There is disclosed a centrifuge blood processing system further selectively controlling a controller in response to either distinguished phase boundaries in an observation region or in response to fluid composition in an outflow passage.

It is disclosed that computational apparatus selects control based on the phase boundaries during transient changes in fluid conditions in a separation chamber.

In addition, computational apparatus may select control based on fluid composition in an outflow passage during relatively steady-state flow conditions in a separation chamber.

It is also a feature of the invention that computational apparatus, may distinguish an average light intensity in a viewing region of an outflow passage. The average light intensity may be a median value selected from a set of discrete light intensity measurements. The discrete light intensity measurements may be a rolling set formed by deleting the oldest light intensity measurement from the set and by adding a new light intensity measurement to the set.

It is to be understood that both the foregoing general description and the following detailed description are exemplary, and are intended to provide further explanation of the invention as claimed.
FIG. 1 is a partial perspective, schematic view of a blood processing centrifuge apparatus including a fluid chamber.
FIG. 2 is a partial perspective, schematic view of the centrifuge apparatus and a control camera.
FIG. 3 is a partial cross-sectional view of blood processing apparatus of Fig. 2, including the fluid chamber of FIG. 1.
FIG. 4 is a partial cross-sectional, schematic view of a portion of a separation vessel and the fluid chamber mounted on a centrifuge rotor of FIG. 1.
FIG. 5 is a plan view of a separation chamber of the separation vessel of Figure 4.
FIG. 6 is a graphic representation of steps for image processing according to the present invention.
FIG. 7 shows the relationship of FIG. 7-a through FIG. 7-d, which illustrate a general program for controlling a blood processing centrifuge apparatus.
FIG. 8 illustrates a program for acquisition of two measures of the collect port intensity, the arithmetic average A and the median B.
FIG. 9 illustrates a software state machine for the blood processing apparatus.
FIG. 10 shows a program for combining the arithmetic average A and the median B of the collect port intensity.
FIG. 11 illustrates the effect of control using the average and the median of the collect port intensity according to FIG. 8 and FIG. 10.
FIG. 12 is a table associating preferred optical interface control with collection states of Fig. 9.

The present invention preferably comprises a blood processing apparatus having a camera control system, as disclosed in U.S. Patent applications 10/884,877 and 10/905,353. It may also be practiced with a TRIMA® blood component centrifuge manufactured by Gambro BCT, Inc. of Colorado or, alternatively, with a COBE® SPECTRA™ single-stage blood component centrifuge also manufactured by Gambro BCT, Inc. Both the TRIMA ® and the SPECTRA™ centrifuges incorporate a one-omega/two-omega sealless tubing connection as disclosed in U.S. Patent No. 4,425,112 to Ito. The SPECTRA™ centrifuge also uses a single-stage blood component separation channel substantially as disclosed in U.S. Patent No. 4,094,461 two Kellogg et al. and U.S. Patent No. 4,647,279 to Mulzet et al. The invention could also be practiced with a TRIMA® or TRIMA ACCEL® centrifugal separation system or other types of centrifugal separator. The method of the invention is described in connection with the aforementioned blood processing apparatus and camera control system for purposes of discussion only, and this is not intended to limit the invention in any sense.

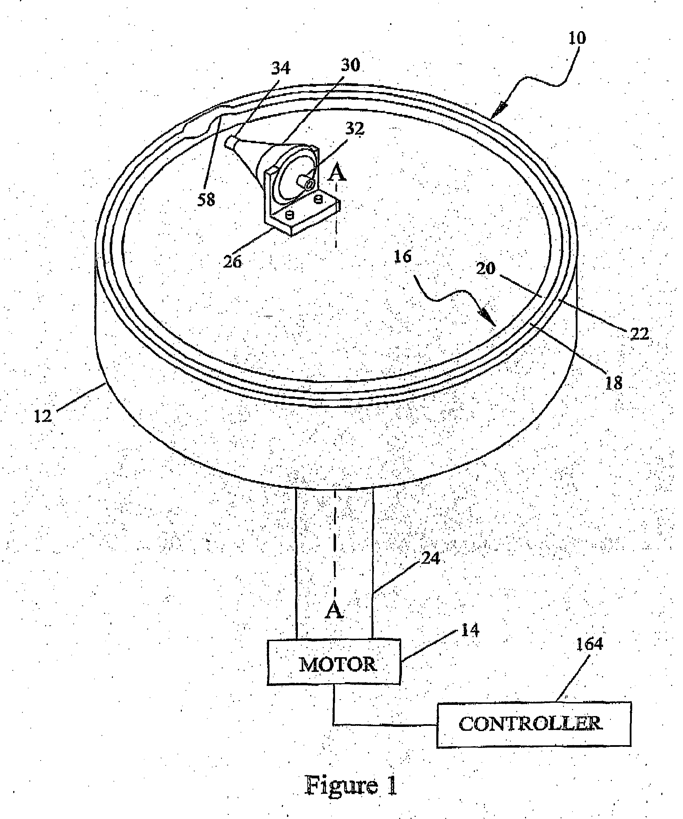

As embodied herein and illustrated in FIG. 1, a centrifuge apparatus 10 has a centrifuge rotor 12 coupled to a motor 14 so that the centrifuge rotor 12 rotates about its axis of rotation A-A. The motor 14 is coupled to the rotor 12 directly or indirectly through a shaft 24 connected to the rotor 12. Alternately, the shaft 24 may be coupled to the motor 14 through a gearing transmission (not shown).

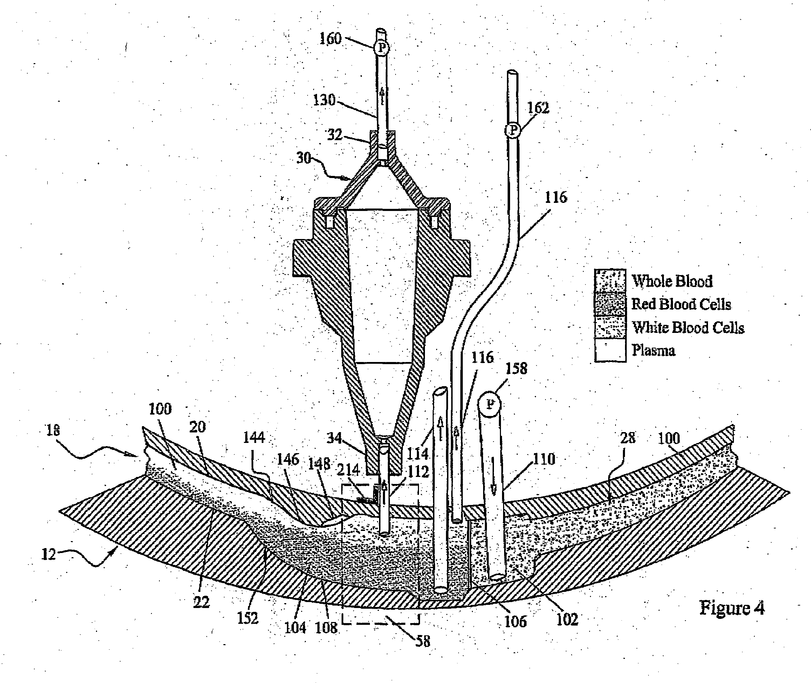

The rotor 12 has a retainer 16 including a passageway or annular groove 18 having an open upper surface adapted to receive a separation vessel 28, shown in pertinent part in FIG. 4. The groove 18 completely surrounds the rotor's axis of rotation A-A and is bounded by an inner wall 20 and an outer wall 22 spaced apart from one another to define the groove 18. Although the groove 18 shown in FIG. 1 completely surrounds the axis of rotation A-A, the groove could partially surround the axis A-A if the separation vessel is not annular. Preferably, a substantial portion of the groove 18 has a constant radius of curvature about the axis of rotation A-A and is positioned at a maximum possible radial distance on the rotor 12. This shape ensures that substances separated in the separation vessel 28 undergo relatively constant centrifugal forces as they pass from an inlet portion to an outlet portion of the separation vessel 28.

As shown in FIG. 1, a bracket 26 is provided on a top surface of the rotor 12. The bracket 26 releasably holds a fluid chamber 30 on the rotor 12 so that an outlet 32 of the fluid chamber 30 is positioned closer to the axis of rotation A-A than an inlet 34 of the fluid chamber 30. The bracket 26 preferably orients the fluid chamber 30 on the rotor 12 with a longitudinal axis of the fluid chamber 30 in a plane transverse to the rotor's axis of rotation A-A. In addition, the bracket 26 is preferably arranged to hold the fluid chamber 30 on the rotor 12 with the fluid chamber outlet 32 facing the axis of rotation A-A. Although the fluid chamber 30 is shown on a top surface of the rotor 12, the fluid chamber 30 could also be secured to the rotor 12 at alternate locations, such beneath the top surface of the rotor 12.

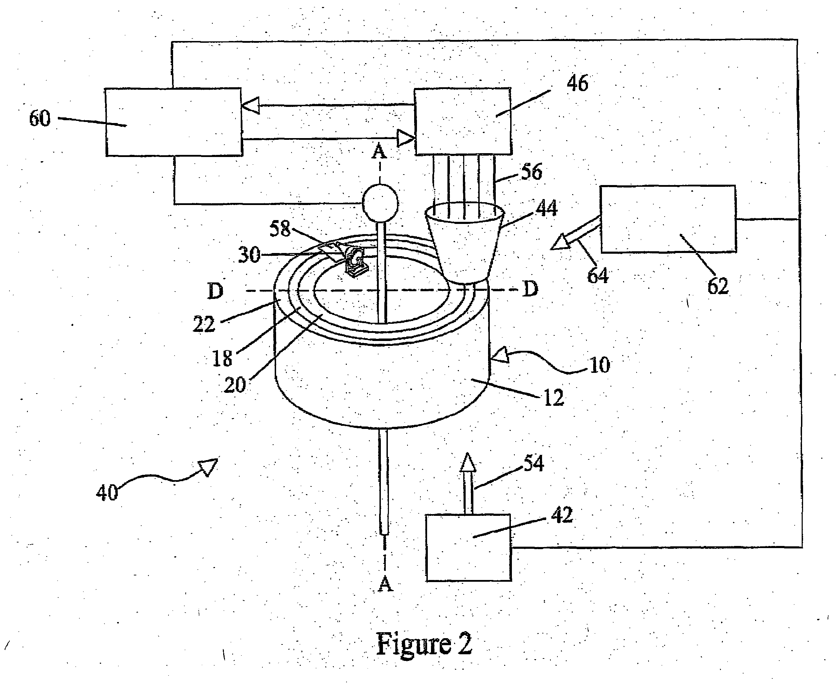

Figure 2 schematically illustrates an exemplary embodiment of an optical monitoring system 40 capable of measuring a distribution of scattered and/or transmitted light intensities corresponding to patterns of light originating from an observation region on the separation vessel 28. The monitoring system 40 comprises light source 42, light collection element 44, and detector 46. Light source 42 is in optical communication with the centrifuge apparatus 10 comprising rotor 12, which rotates about central rotation axis A-A. Rotation about central rotation axis A-A results in separation of a blood sample in the separation vessel 28 into discrete blood components along a plurality of rotating separation axes oriented orthogonal to the central rotation axis A-A.

Light source 42 provides incident light beam 54, which stroboscopically illuminates an observation region 58 when the observation region 58 passes under the light collection element 44. Light source 42 is capable of generating an incident light beam, a portion of which is transmitted through at least one blood component undergoing separation in separation vessel 28. At least a portion of scattered and/or transmitted light 56 from the observation region 58 is collected by light collection element 44. Light collection element 44 is capable of directing at least a portion of the collected light 56 onto detector 46. The detector 46 detects patterns of scattered and/or transmitted light 56 from the observation region. Optionally, the observation region 58 may also be illuminated by an upper light source 62, which is positioned on the same side of the separation chamber as the light collection element 44 and detector 46. Upper light source 62 is positioned such that it generates an incident beam 64, which is scattered by the blood sample and/or centrifuge. A portion of the light from upper light source 62 is collected by light collection element 44 and detected by detector 46, thereby measuring a distribution of scattered and/or transmitted light intensities.

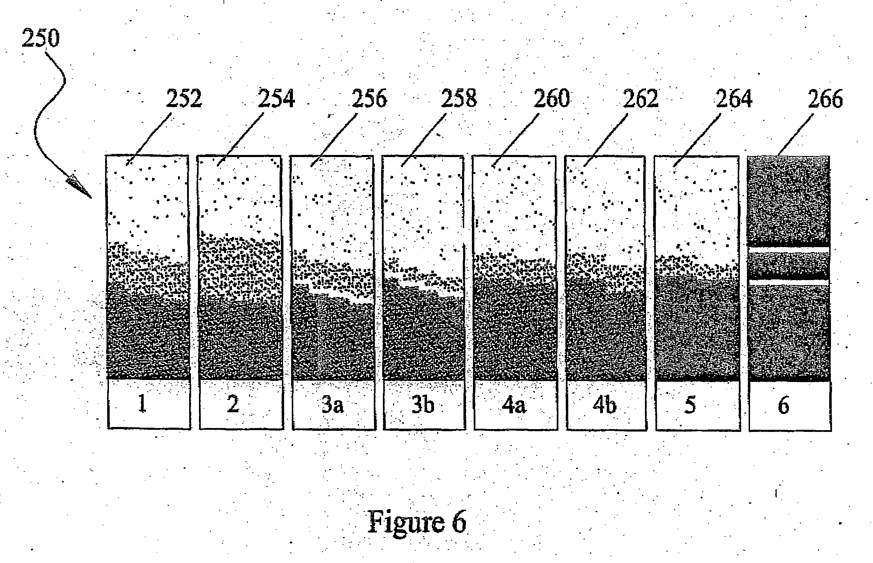

Distributions of scattered and/or transmitted light intensities comprise images corresponding to patterns of light originating from the observation region 58. The images may be monochrome images, which provide a measurement of the brightness of separated blood components along the separation axis. Alternatively, the images may be color images, which provide a measurement of the colors of separated blood components along the separation axis. Observation region 58 is positioned on a portion of the density centrifuge 10, preferably on the separation vessel 28. The fluid chamber 30 may also be an observation region, as explained below. In the exemplary embodiment illustrated in Figure 6, separated blood components and phase boundaries between optically differentiable blood components are viewable in observation region 58.

Detector 46 is also capable of generating output signals corresponding to the measured distributions of scattered and/or transmitted light intensities and/or images. The detector 46 is operationally connected to a device controller 60 capable of receiving the output signals. Device controller 60 displays the measured intensity distributions, stores the measured intensity distributions, processes measured intensity distributions in real time, transmits control signals to various optical and mechanical components of the monitoring system and centrifuge or any combination of these. Device controller 60 is operationally connected to centrifuge apparatus 10 and is capable of adjusting selected operating conditions of the centrifuge apparatus, such as the flow rates of cellular and non-cellular components out of the separation vessel 28 or fluid chamber 30, the position of one or more phase boundaries, rotational velocity of the rotor about central rotation axis A-A, the infusion of anticoagulation agents or other blood processing agents to the blood sample, or any combination of these.

Device controller 60 can also be operationally connected to light source 42 and/or upper light source 62. Device controller 60 and/or detector 46 are capable of generating output signals for controlling illumination conditions. For example, output signals from the detector 46 can be used to control the timing of illumination pulses, illumination intensities, the distribution of illumination wavelengths and/or position of light source 42 and/or upper light source 62. Device controller 60 and detector 46 are in two-way communication, and the device controller sends control signals to detector 46 to selectively adjust detector exposure time, detector gain and to switch between monochrome and color imaging.

Light sources comprise light emitting diode sources capable of generating one or more incident beams for illuminating an observation region on the centrifuge. A plurality of lamps may be positioned to illuminate a single side or multiple sides of the centrifuge apparatus 10. Light emitting diodes and arrays of light emitting diode light sources are preferred for some applications because they are capable of generating precisely timed illumination pulses. Preferred light sources generate an incident light beam having a substantially uniform intensity, and a selected wavelength range.

The optical monitoring system comprises a plurality of light sources, each capable of generating an incident light beam having a different wavelength range, for example, a combination of any of the following: white light source, red light source, green light source, blue light source and infra red light source. Use of a combination of light sources having different wavelength ranges is beneficial for discriminating and characterizing separated blood fractions because absorption constants and scattering coefficients of cellular and non-cellular components of blood vary with wavelength. For example, a component containing red blood cells is easily distinguished from platelet-enriched plasma by illumination with light having wavelengths selected over the range of about 500 nm to about 600 nm, because the red blood cell component absorbs light over this wavelength significantly more strongly that the platelet-enriched plasma component. In addition, use of multiple colored light sources provides a means of characterizing the white blood cell type in an extracted blood component. As different white blood cell types have different absorption and scattering cross sections at different wavelengths, monitoring transmitted and/or scattered light from a white cell-containing blood component provides a means of distinguishing the various white blood cell types in a blood component and quantifying the abundance of each cell-type.

The light sources provide a continuous incident light beam or a pulsed incident light beam. Pulsed light sources are switched on and off synchronously with the rotation of the rotor to illuminate an observation region having a substantially fixed position on the rotor. Alternatively, pulsed light sources of the present invention can be configured such that they can be switched on and off at different angular positions, synchronous with the rotation of the rotor, illuminating different observation regions for each full rotation. This alternative embodiment provides a method of selectively adjusting the location of the observation region and, thereby, probing different regions of the separation chamber or of the fluid chamber 30. Triggering of illumination pulses may be based on the rotational speed of the centrifuge or on the angular position of the separation chamber or the fluid chamber 30 as detected by optical or electronic methods well known in the art. Triggering may be provided by trigger pulses generated by the device controller 60 and/or detector 46.

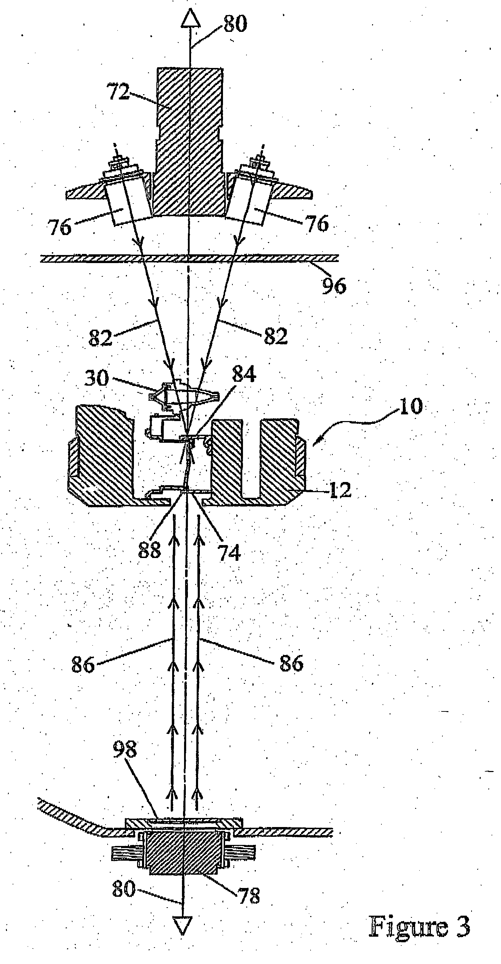

Figure 3 is a cutaway view corresponding to cutaway of the optical monitoring system 40. The illustrated optical monitoring system 40 comprises CCD camera 72 (CMOS, APS or other cameras could also be used) equipped with a fixed focus lens system (corresponding to the light collection element 44 and detector 46), an optical cell 74 (corresponding to the observation region 58), an upper LED light source 76 (corresponding to the upper light source 62), and a bottom pulsed LED light source 78 (corresponding to the light source 42). As illustrated in Figure 3, CCD camera 72 is in optical communication with optical cell 74 and positioned to intersect optical axis 80. Upper LED light source 76 is in optical communication with optical cell 74 and is positioned such that it is capable of directing a plurality of collimated upper light beams 82, propagating along propagation axes that intersect optical axis 80, onto the top side 84 of optical cell 74. Bottom pulsed LED light source 78 is also in optical communication with optical cell 74 and is positioned such that it is capable of directing a plurality of collimated bottom light beams 86, propagating along optical axis 80, onto the bottom side 88 of optical cell 74.

CCD camera 72 may be positioned such that the focal plane of the fixed focus lens system is substantially co-planar with selected optical surfaces of optical cell 74, such as optical surfaces corresponding to an interface monitoring region, calibration markers, one or more extraction ports and one or more inlets. The CCD camera 72 is separated from the center of the fixed focus lens system by a distance along optical axis 80 such that an image corresponding to selected optical surfaces of optical cell 74 is provided on the sensing surface of the CCD camera. This optical configuration allows distributions of light intensities comprising images of rotating optical cell 74 or of fluid chamber 30 to be measured and analyzed in real time.

Referring to Figure 3, first transparent plate 96 is provided between CCD camera 72 and optical cell 74, and second transparent plate 98 is provided between bottom LED light source 78 and optical cell 74. First and second transparent plates 96 and 98 physically isolate CCD camera 72, upper LED light source 76 and bottom LED light source 78 from optical cell 74 so that these components will not contact a sample undergoing processing in the event of sample leakage from the separation chamber. In addition, first and second transparent plates 96 and 98 minimize degradation of CCD camera 72, upper LED light source 76 and bottom LED light source 78 due to unwanted deposition of dust and other contaminants that can be introduced to the system upon rotation of the separation chamber and filler. Further, first and second transparent plates 96 and 98 also allow a user to optimize the alignment of the camera, upper LED light source and bottom LED light source without exposure to a blood sample in the separation chamber. First and second transparent plates 96 and 98 can comprise any material capable of transmitting at least a portion of upper and bottom illumination light beams 82 and 86. Exemplary materials for first and second transparent plates 96 and 98 include, but are not limited to, glasses such as optical quality scratch resistant glass, transparent polymeric materials such as transparent plastics, quartz and inorganic salts.

FIG. 4 schematically illustrates a portion of the separation vessel 28 and fluid chamber 30 mounted on the rotor 12. The separation vessel 28 has a generally annular flow path 100 and includes an inlet portion 102 and outlet portion 104. A wall 106 prevents substances from passing directly between the inlet and outlet portions 102 and 104 without first flowing around the generally annular flow path 100 (e.g., counterclockwise in FIG. 4).

A radial outer wall 108 of the separation vessel 28 is positioned closer to the axis of rotation A-A in the inlet portion 102 than in the outlet portion 104. During separation of blood components, this arrangement causes formation of a very thin and rapidly advancing red blood cell bed in the separation vessel 28 between the inlet portion 102 and outlet portion 104. The red blood cell bed reduces the amount of blood components required to initiate a separation procedure, and also decreases the number of unnecessary red blood cells in the separation vessel 28. The red blood cell bed substantially limits or prevents platelets from contacting the radial outer wall 108 of the separation vessel 28. This is believed to reduce clumping of platelets caused when platelets contact structural components of centrifugal separation devices.

The inlet portion 102 includes an inflow tube 110 for conveying a fluid to be separated, such as whole blood, into the separation vessel 28. During a separation procedure, substances entering the inlet portion 102 follow the flow path 100 and stratify according to differences in density in response to rotation of the rotor 12. The outlet portion 104 includes first, second, and third outlet lines 112, 114, 116 for removing separated substances from the separation vessel 28. Preferably, each of the components separated in the vessel 28 is collected and removed in only one area of the vessel 28, namely the outlet portion 104. In addition, the separation vessel 28 preferably includes a substantially constant radius except in the region of the outlet portion 104 where the outer wall of the outlet portion 104 is preferably positioned farther away from the axis of rotation A-A to allow for outlet ports of the lines 112, 114, and 116 to be positioned at different radial distances and to create a collection pool with greater depth for the high density red blood cells. The outlet port of line 114 is farther from the axis of rotation A-A than the other ports to remove higher density components, such as red blood cells. The port of line 116 is located closer to the axis of rotation A-A than the other ports to remove the least dense components separated in the separation vessel 28, such as plasma. The first line 112 collects intermediate density components and, optionally, some of the lower density components. The second and third lines 114 and 116 are positioned downstream from first line 112 to collect the high and low density components.

The positions of the interfaces are controlled by the CCD camera 72 monitoring the position of the interface and controlling-flow of liquid and/or particles in response to the monitored position. Further details concerning the structure and operation of the separation vessel 28 are described in US Patent Application 10/884,877 and also in U.S. Patent No. 4,094,461 to Kellogg et al. and U.S. Patent No. 4,647,279 to Mulzet et al.

A ridge 144 extends from the inner wall 20 of the groove 18 toward the outer wall 22 of the groove 18. When the separation vessel 28 is loaded in the groove 18, the ridge 144 deforms semi-rigid or flexible material in the outlet portion 104 of the separation vessel 28 to form a trap dam 146 in the separation vessel 28, upstream from the first line 112. The trap dam 146 extends away from the axis of rotation A-A to trap a portion of lower density substances, such as priming fluid and/or plasma, along an inner portion of the separation vessel 28 located upstream the trap dam 146. These trapped substances help convey platelets to the outlet portion 104 and first line 112 by increasing plasma flow velocities next to the layer of red blood cells in the separation vessel 28 to scrub platelets toward the outlet portion 104. A downstream portion 148 of the trap dam 146 has a relatively gradual slope extending in the downstream direction toward the axis of rotation A-A, which limits the number of platelets (intermediate density components) that become re-entrained (mixed) with plasma (lower density components) as plasma flows along the trap dam 146. In addition, the gradual slope of the downstream portion 148 reduces the number of platelets that accumulate in the separation vessel 28 before reaching the first collection port 120.

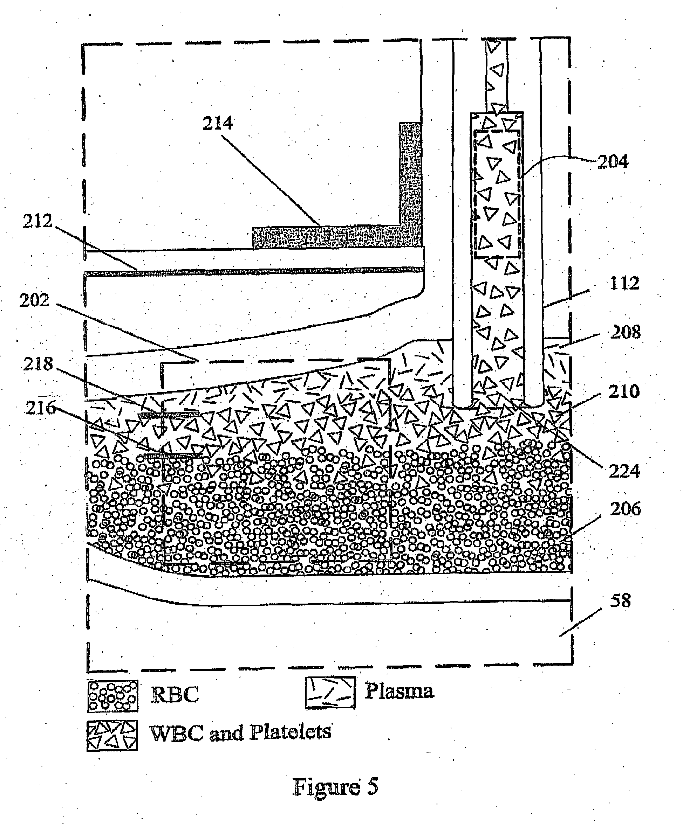

The camera 44 is generally focused on the separation vessel and stroboscopic illumination allows an observation region 58 around the first, second, and third lines 112, 114, and 116 to be observed. Using information gathered through the camera, the controller 60 regulates the position of interfaces between various blood components, such as plasma, buffy coat (containing monocytes and/or white blood cells and platelets) and red blood cells by controlling the pumps 158, 160, and 162. Figure 5 shows an image of the observation region 58 generated by the methods of US patent application 10/884,877 corresponding to the separation of a human blood sample and extraction of a separated white blood cell-containing blood component. The observation region 58 shown in Figure 5 includes a phase boundary monitoring region 202 and an extraction or collect port monitoring region 204. Visible in phase boundary monitoring region 202 are a red blood cell component 206, a plasma component 208 and a mixed-phase buffy coat layer 210, which has both white blood cells and platelets. Several calibration markers are also apparent in the image in Figure 5. Near an edge 212 of the optical cell is an L-shaped calibration marker or optical reference 214 for determining the absolute position of phase boundaries between optically differentiable blood components. The inner edge of the optical reference 214 is used to indicate the positions and physical dimensions of the phase boundary monitoring region 202 and the white blood cell collect port monitoring region 204. The physical dimension may be determined by adjusting the optics to within a selected range and then configuring the software with a parameter to convert pixels to microns. Alternatively, the thickness of the optical reference, usually about 1 mm, could be used. Light intensities transmitted through the phase boundary monitoring region 202 are acquired as a function of time and analyzed in real time to provide measurements of the position of the phase boundary 216 between red blood cell component 206 and buffy coat layer 210 and the phase boundary 218 between the buffy coat layer 210 and plasma component 208. All boundary layer positions are measured relative to the edge of the optical reference 214.

Collect port monitoring region 204 monitors flow in first line 112 of the optical cell for extracting a blood component, for example, white blood cells. The apparatus responds to changes in detected blood component flow to establish a correct phase boundary level and further responds to changes in observed phase boundaries to maintain a consistent phase boundary level. The system discriminates between a plasma flow condition, a white blood cell flow condition, and a red blood cell flow condition, and can detect pump-induced flow variation in the blood component flow in said collect port measuring area. A plasma signal limit and a red blood cell signal limit may be set and the flow of fluid adjusted based on said limits. The system derives a statistical measure of fluid flow in the collect port measuring area, which may be a moving median of the average value of intensity of pixels in the collect port measuring area.

In this example, first line 112 having orifice 224 is configured to collect white blood cells in the human blood sample and extends a distance along the separation axis such that it terminates proximate to the buffy coat layer in the rotating separation chamber. The two-dimensional distribution of light intensities of light transmitted through the collect port in the collect port monitoring region 204 depends on the concentration, and spatial distribution and cell-type of cellular material exiting the separation chamber. Light intensities transmitted through the collect port monitoring region 204 are acquired as a function of time and analyzed to characterize the composition and flux of cellular material out of the separation chamber. As cellular materials, such as white blood cells and red blood cells, absorb and scatter light from the light sources, passage of cellular material through the extraction port decreases the observed light intensities.

Referring again to FIG. 4, the outer wall 22 of the groove 18 preferably includes a gradual sloped portion 152 facing the ridge 144 in the inner wall 20. When the separation vessel 28 is loaded in the groove 18, the gradual sloped portion 152 deforms semi-rigid or flexible material in the outlet portion 104 of the separation vessel 28 to form a relatively smooth and gradual sloped segment in a region of the vessel 28 across from the trap dam 146, which slopes gradually away from the axis of rotation A-A to increase the thickness of a layer of high-density fluid components, such as red blood cells, formed across from the trap dam 146.

The first collection line 112 is connected to the fluid chamber inlet 34 to pass the intermediate density components into the fluid chamber 30. Components initially separated in the separation vessel 28 are further separated in the fluid chamber 30. For example, white blood cells could be separated from plasma and platelets in the fluid chamber 30. This further separation preferably takes place by forming a saturated fluidized bed of particles, such as white blood cells, in the fluid chamber 30. The fluid chamber 30 may be formed of a transparent or translucent co-polyester plastic, such as PETG, to allow viewing of the contents within the chamber interior with the aid of the camera during a separation procedure.

As schematically shown in FIG. 4, a plurality of pumps 158, 160, and 162 are provided for adding and removing substances to and from the separation vessel 28 and fluid chamber 30. An inflow pump 158 is coupled to the inflow line 110 to supply the substance to be separated, such as whole blood, to the inlet portion 102. In addition, a first collection pump 160 is flow coupled to the outflow tubing 130 connected to the fluid chamber outlet 32, and a second collection pump 162 is flow coupled to the third collection line 116. The first collection pump 160 draws liquid and particles from the fluid chamber outlet 32 and causes liquid and particles to enter the fluid chamber 30 via the fluid chamber inlet 34. The second collection pump 162, on the other hand, removes primarily low-density substances from the separation vessel 28 via the third line 116.

The pumps 158, 160, and 162 are peristaltic pumps or impeller pumps configured to prevent significant damage to blood components. However, any fluid pumping or drawing device may be provided. In an alternative embodiment (not shown), the first collection pump 160 may be fluidly connected to the fluid chamber inlet 34 to directly move substances into and through the fluid chamber 30. In addition, the pumps 158, 160, and 162 may be mounted at any convenient location. The inflow pump 150 and the first collection pump 160 may be configured so that substances do not bypass these pumps when they are paused. For example, when the first collection pump 160 is temporarily paused, substances pumped by the second collection pump 162 flow into the fluid chamber outlet 32 rather than bypassing the pump 160 and flowing in the opposite direction.

The apparatus 10 further includes a controller 164 (Fig. 1) connected to the motor 14 to control rotational speed of the rotor 12. The controller 164 is connected to the pumps 158, 160, and 162 to control the flow rate of substances flowing to and from the separation vessel 28 and the fluid chamber 30. The controller 164 controls the operation and flow rate of the pumps 158, 160, 162 to permit the temporary purging of the fluid chamber 30. The controller 164 may include a computer having programmed instructions provided by a ROM or RAM as is commonly known in the art. The controller 164 may vary the rotational speed of the centrifuge rotor 12 by regulating frequency, current, or voltage of the electricity applied to the motor 14. Alternatively, the rotational speed can be varied by shifting the arrangement of a transmission (not shown), such as by changing gearing to alter a rotational coupling between the motor 14 and rotor 12. The controller 164 may receive input from a rotational speed detector (not shown) to constantly monitor the rotation speed of the rotor.

After loading the separation vessel 28 and fluid chamber 30 on the rotor 12, the separation vessel 28 and chamber 30 are initially primed with a low density fluid medium, such as air, saline solution, plasma, or another fluid substance having a density less than or equal to the density of liquid plasma. Alternatively, the priming fluid is whole blood itself. This priming fluid allows for efficient establishment of a saturated fluidized bed of platelets within the fluid chamber 30. When saline solution is used, the pump 158 pumps this priming fluid through the inflow line 110 and into the separation vessel 28 via the inlet line 110. The saline solution flows from the inlet portion 102 to the outlet portion 104 (counterclockwise in FIG. 4) and through the fluid chamber 30 when the controller 164 activates the pump 160. Controller 164 also initiates operation of the motor 14 to rotate the centrifuge rotor 12, separation vessel 28, and fluid chamber 30 about the axis of rotation A-A. During rotation, twisting of lines 110, 112, 114, 116, and 130 is prevented by a sealless one-omega/two-omega tubing connection as is known in the art and described in above-mentioned U.S. Patent No. 4,425,112.

As the separation vessel 28 rotates, a portion of the priming fluid (blood or saline solution) becomes trapped upstream from the trap dam 146 and forms a dome of priming fluid (plasma or saline solution) along an inner wall of the separation vessel 28 upstream from the trap dam 146. After the apparatus 10 is primed, and as the rotor 12 rotates, whole blood or blood components are introduced into the separation vessel 28. When whole blood is used, the whole blood can be added to the separation vessel 28 by transferring the blood directly from a donor or patient through inflow line 110. In the alternative, the blood may be transferred from a container, such as a blood bag, to inflow line 110.

The blood within the separation vessel 28 is subjected to centrifugal force causing components of the blood components to separate. The components of whole blood stratify in order of decreasing density as follows: (1) red blood cells, (2) white blood cells, (3) platelets, and (4) plasma. The controller 164 regulates the rotational speed of the centrifuge rotor 12 to ensure that this particle stratification takes place. A layer of red blood cells (high density component(s)) forms along the outer wall of the separation vessel 28 and a layer of plasma (lower density component(s)) forms along the inner wall of the separation vessel 28. Between these two layers, the intermediate density platelets and white blood cells (intermediate density components) form a buffy coat layer. This separation takes place while the components flow from the inlet portion 102 to the outlet portion 104. Preferably, the radius of the flow path 100 between the inlet and outlet portions 102 and 104 is substantially constant to maintain a steady red blood cell bed in the outlet portion 104 even if flow changes occur.

In the outlet portion 104, platelet poor plasma flows through the third line 116. These relatively low-density substances are pumped by the second collection pump 162 through the third collection line 116. Red blood cells are removed via the second line 114. The red blood cells flow through the second collection line 114 and can then be collected and optionally recombined with other blood components or further separated. Alternately, these removed blood components may be re-infused into a donor or patient.

Accumulated platelets are removed via the first collection line 112 along with some of the white blood cells and plasma. As the platelets, plasma, white blood cells, and possibly a small number of red blood cells pass through the first collection-line 112, these components flow into the fluid chamber 30, filled with the priming fluid, so that a saturated fluidized particle bed may be formed. The platelets flow toward the first collection line 112. The priming fluid along the inner walls of the separation vessel 28 reduces the effective passageway volume and area in the separation vessel 28 and thereby decreases the amount of blood initially required to prime the system in a separation process. The reduced volume and area also induces higher plasma and platelet velocities next to the stratified layer of red blood cells, in particular, to "scrub" platelets toward the first collection line 112. The rapid conveyance of platelets increases the efficiency of collection.

The controller 164 maintains the rotation speed of the rotor 12 within a predetermined rotational speed range to facilitate formation of this saturated fluidized bed. In addition, the controller 164 regulates the pump 160 to convey at least the plasma, platelets, and white blood cells at a predetermined flow rate through the first collection line 112 and into the inlet 34 of the fluid chamber 30. These flowing blood components displace the priming fluid from the fluid chamber 30. When the platelet and white blood cell particles enter the fluid chamber 30, they are subjected to two opposing forces. Plasma flowing through the fluid chamber 30 with the aid of pump 160 establishes a first viscous drag force when plasma flowing through the fluid chamber 30 urges the particles toward the outlet 32. A second centrifugal force created by rotation of the rotor 12 and fluid chamber 30 acts to urge the particles toward the inlet 34.

The controller 164 regulates the rotational speed of the rotor 12 and the flow rate of the pump 160 to collect platelets and white blood cells in the fluid chamber 30. As plasma flows through the fluid chamber 30, the flow velocity of the plasma decreases and reaches a minimum as the plasma flow approaches the maximum cross-sectional area of the fluid chamber 30. Because the rotating centrifuge rotor 12 creates a sufficient gravitational field in the fluid chamber 30, the platelets accumulate near the maximum cross-sectional area of the chamber 30, rather than flowing from the chamber 30 with the plasma. The white blood cells accumulate somewhat radially outward from the maximum cross-sectional area of the chamber 30. However, density inversion tends to mix these particles slightly during this initial establishment of the saturated fluidized particle bed.

The fluid chamber 30 is configured to allow cyclic collection of selected particles, such as white blood cells, followed by efficient evacuation of the cells into a collection bag. In contrast to other chamber designs for forming saturated fluidized beds, the fluid chamber described herein has particular application for the automated collection of blood components in that a bolus of cells having selected characteristics can be collected in the fluid chamber 30 and then flushed with low density fluid into a collection bag and these steps can be repeated multiple times, allowing a larger quantity of the selected cells to be collected from the donor or patient while reducing the amount of time necessary for the donation process. Collection of cells in the fluid chamber can be monitored by the camera 72 and the device controller 60. When a selected quantity of cells have been collected in the fluid chamber 30, the flow of plasma through the chamber can be increased and the collected cells can be washed out of the chamber and directed into a collection bag.

In a high-speed centrifuge for separating blood components, control of the interface between blood components presents significant control problems. The present apparatus controls the interface location by measuring light intensity in the collect port monitoring region 204 in the collect port by detecting the presence or absence of RBC's in the collect port, and by monitoring the interface 216 or 218 in the phase boundary or interface monitoring region 202. The light intensity in the collect port can be measured by both an average value over a relatively brief period of time or by a median value over a longer period of time or by a combination of both measurements. The location of the interface is detected by a series of image processing steps, which allow the apparatus to recognize a boundary or interface despite the high speed of the centrifuge rotor, the stroboscopic light used for observation, and the limitations of data processing time caused by the need for real-time response to changes in the interface location. Monitoring the interface in the interface monitoring region 202 allows the apparatus to control the location of the interface with stability. The image processing steps for controlling the interface are represented in Fig. 6, which shows a series 250 of images representing the character of data derived from a view of the interface. It will be understood that these images are not displayed to an operator but rather illustrate the condition of image data in a computer. Interface detection in the monitoring region 202 may comprise the steps of spoiling 252 the image, diffusing 254 the image, edge detection 256 and 258, edge linking 260, 262, region-based confirmation 264 and interface calculation 266. These image processing steps will be described herein in connection with general flow chart representations for clarity. It will be understood that one skilled in the art would implement the programs in a selected programming language, such as C++ for example, but the programs could also be implemented in machine language, in firmware, or in dedicated circuitry without departing from the teachings set forth herein. "Spoiling" 252 the image reduces the number of pixels to be examined preferentially on orthogonal axis oriented with respect to the expected location of the interface or phase boundary. For example, if the pixels of the image are oriented in rows parallel to the interface and in columns perpendicular to the interface, the software might sample every third pixel along rows and every tenth pixel along columns. This reduces the number of pixels to be processed, while retaining sufficient detail in a preferred direction to detect changes in the interface location. "Diffusing" 254 the image smoothes out small oscillations in the interface boundary, making the location of the interface more distinct. "Edge detection" 256, 258 computes the rate of change in pixel intensity (that is, the derivative of the pixel intensity) as a function of distance in x (parallel to rotation) and y (perpendicular to rotation or radially with respect to the centrifuge) directions. Locations where the derivatives reach maxima indicate sharp intensity changes between pixels, which may represent an interface. "Edge linking" 260, 262 connects adjacent maxima. A chain of such connected maxima is identified as an edge of an interface if the chain is sufficiently long. The length may be predetermined empirically. Short chains are ignored as waves or other flow phenomenon. To confirm that the boundaries have actually been detected, the software uses the potential boundaries to form regions on either sides of the boundary and determines the average intensity of pixels in each region. "Region-based confirmation" 264 creates a pseudo image of the regions that qualify as distinct, that is, having at least a predetermined difference in average intensity, and shades each region differently. "Final edge calculation" 266 uses the points where the shade changes in the pseudo image, averages the y (radial) of these points and recognizes this average radial location as the true interface position.

This image processing is fast enough to respond to the high speed of the centrifuge in real time, yet sufficiently robust to detect subtle changes in the location of the interface or interfaces such that rotor speed or pump speed can be changed to correct and control the interface location. Responding to changes in intensity in the collect port monitoring region 204 would not be rapid enough to maintain the quality of blood product being collected.

Collect port measuring of intensity in the collect port monitoring region 204 allows measurement of cellular material leaving through the collect port in real time. Statistical measures may be used to such parameters as the hematocrit of collected blood product, allowing for more accurate collection of a desired type of product. The present apparatus selects between image processing in of the phase boundary and intensity measurements in the collect port region to control fluid flow in the blood processing apparatus.

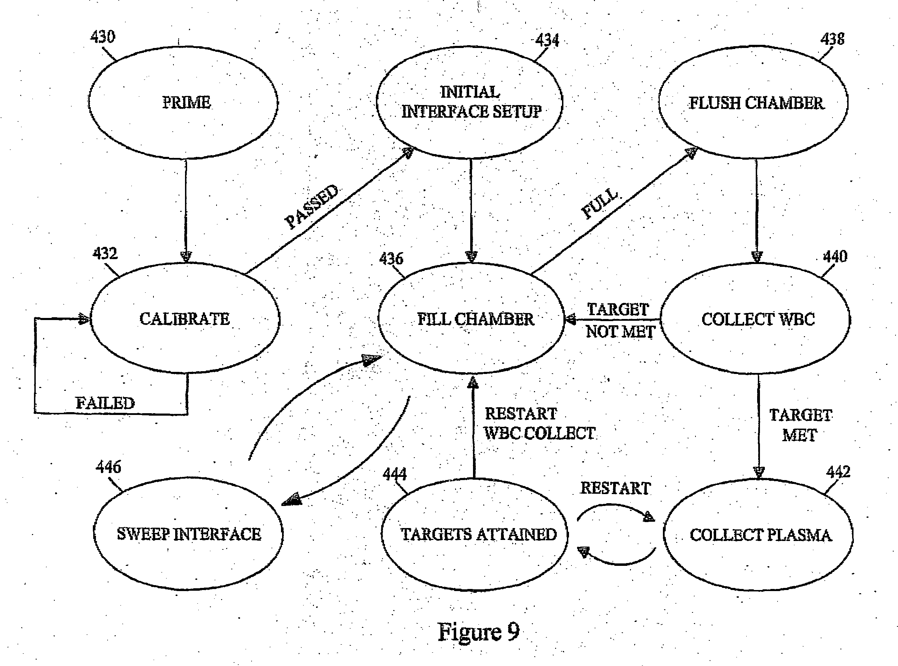

As illustrated in Figure 9, the blood processing apparatus passes control through several states in the process of collecting selected blood components such as red blood cells, white blood cells, or plasma. Initially, the apparatus is primed 430 and then tries to calibrate 432 the light sensing apparatus, that is, the camera and lights, for the existing operating conditions. The apparatus would report if the calibration procedure failed or if the sensed conditions were within nominal limits. Initial set up procedures 434 would establish the initial interface. This process may proceed under the control of a selected optical sensing protocol, for example, the interface phase boundary control. Depending on the procedure selected for operation, the apparatus may collect white blood cells or other blood components in an LRS (leukocyte reduction) chamber, that is, fill 436 the LRS chamber. When the chamber is full, the apparatus may flush 438 the collected cells out of the LRS chamber into a collection bag. White blood cells, for example, may be collected 440, and control of the apparatus may be periodically returned to the fill state 436, until a white blood cell collection target is attained. Plasma may then be collected 442 until desired targets are attained 444. The apparatus may return to the fill state 436 and again flush the filled 438 chamber to collect white blood cells 440 or plasma 442. During the fill state 436, the apparatus may, from time to time, run a mid run interface setup, or sweep 446 of the interface, controlling the interface between blood component phases. As explained below, this process may comprise selecting one of the optical sensing protocols, for example, sensing light intensity in the outflow port and controlling rate and changes in rate of control pumps within certain parameters.

In the sweep interface 446 sub-process, the phase boundary or interface between red blood cell layer and the buffy coat layer (containing most white blood cells) may be repeatedly raized and lowered to collect white cells in the upper region of the red blood cell layer, even though some red blood cell would be collected at the same time. It is believed that maximizing collection of white blood cells is often a preferred collection choice, and that collection of a small amount of red blood cells can be tolerated.

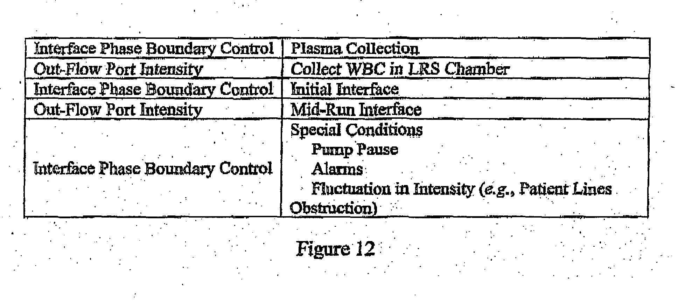

Each of the described states 432, 434, 436, 438, 440, 442, 444, and 446 may be associated with a preferred optical sensing control, as illustrated in the table shown in Figure 12. Thus, for instance, plasma collection 434 may utilize interface phase boundary control based on two-dimensional optical sensing of the location of the phase boundary in the separation chamber. This phase boundary sensing protocol may also be selected for such conditions as establishing the initial interface 434, or for special conditions such as initiated by the operator such as pump pause, or by alarms, large fluctuations in output line intensity, or other situations that cause large changes in operating conditions, such as pump pause, alarms, or fluctuations in line intensity as may be caused by obstructions in the lines. On the other hand, measurement of the optical intensity in the outflow chamber may be used especially where fine control is needed over a relatively long period, such as during white blood cell collection 440, or during interface sweep 446.

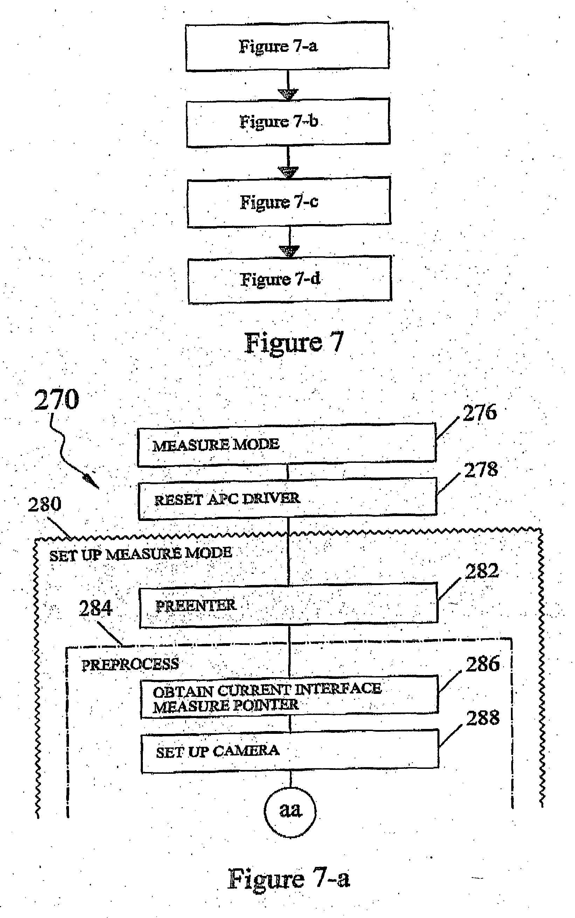

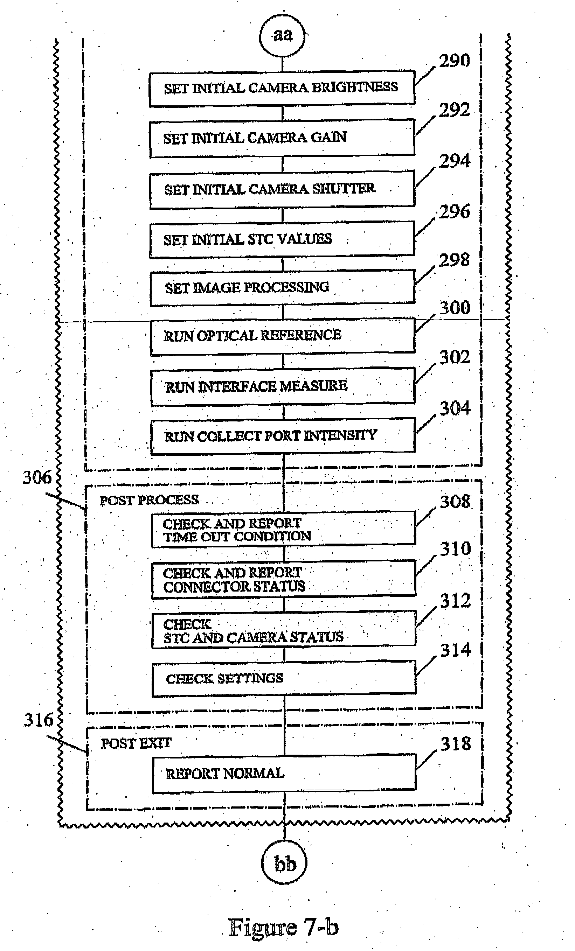

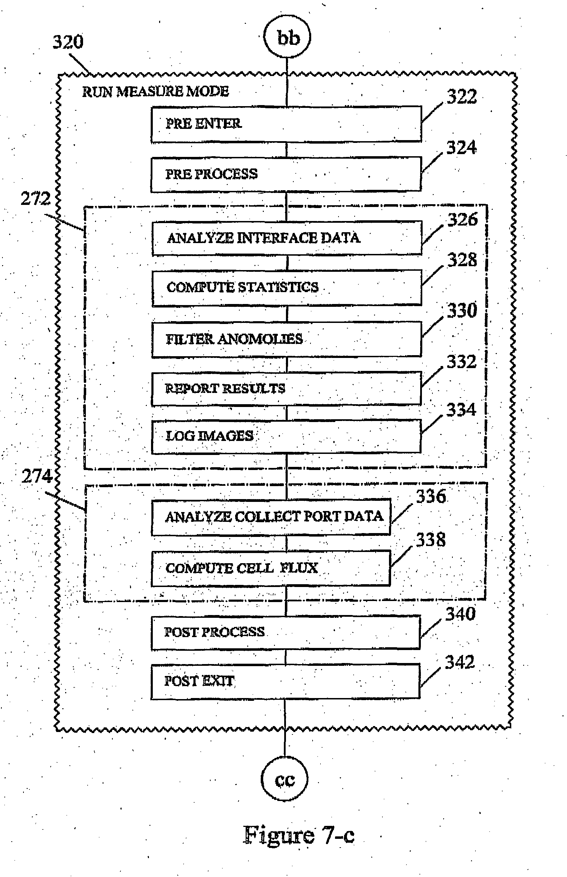

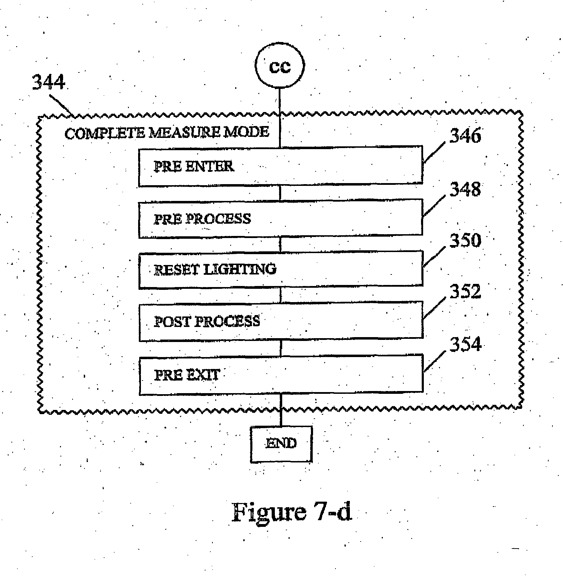

The image processing of the phase boundary is implemented through a measure mode state machine 270 illustrated in Fig. 7. This is more fully described in US Provisional Patent Application the disclosure of which is incorporated herein. The measure mode state machine 270 collects and analyzes data from an interface measurement tool 272 and a collect port intensity tool 274. Raw data from the tools 272, 274 is analyzed and converted into a form that is usable by a control subsystem that controls pumps, rotor and other operating parameters of the aphaeresis machine. At a high level of abstraction, the measure mode 270 is first invoked at a start 276. The program resets 278 the APC (Automated Process Control) driver and enters a measure mode set-up subroutine 280. In the measure mode set-up subroutine 280, the computer performs a pre-enter step 282. The pre-enter step 282 initializes parameters and variables, checks the current time from an internal clock, and checks for error conditions. A pre-process subroutine 284 obtains 286 a current interface measure pointer and sets up 288 the camera to acquire an image. The program sets an initial camera brightness 290, initial camera gain 292, and initial camera shutter speed 294. It also sets initial STC (Synchronous Timing Control) values 296 and image processing parameters 298. Next, the program runs an optical reference 300 to stabilize the optical image with respect to registration markers in the imaging area. It also runs an interface measure 302 to locate the interface or interfaces in the blood under centrifugation and a collect port intensity measure 304 to sense the emitted or reflected light in the area of the collection port.

A post-process subroutine 306 checks and reports 308 any time-out conditions that may occur, as well as connector status 310 and STC and camera status 312. The post-process subroutine further checks settings 314 such as brightness, camera gain, image format or frame rate. A post-exit subroutine 316 reports the status of the set-up measure mode procedure, which will be normal 318 if the apparatus is properly prepared to measure and analyze collected data.

A measure mode subroutine 320 processes acquired data in the interface measurement tool 272 and the collect port intensity tool 274. Initially, a pre-enter step 322 checks that valid pre-conditions exist for running the measure mode subroutine 320 such as checking the existence of the state machine program, initializing variables, and resetting indices preparatory to further data processing. A pre-process 324 then sets pointers and other variables for the data processing, for example, obtaining a current interface measure pointer, updating lighting parameters, selecting conditions for measurement of desired blood components such as plasma or platelets, and checking that both the connector locator and interface measure data are for the same image.

If it is determined that appropriate data is being collected, the interface measurement tool 272 can analyze the optical data to identify the location of an interface between adjacent blood components. First, the interface data is analyzed 326, as will be more fully explained below. From the analyzed data, certain statistical measures may be computed 328, such as mean plasma to buffy coat position, standard deviation, and buffy coat to red blood cell position standard deviation. These measures may be compared to acceptable limits and error log messages generated if they fall outside of acceptable ranges. If no interface has been identified, it may be determined that only plasma or only red blood cells are visible in the interface monitoring region 202. Certain statistics on the interface position may be computed and anomalies filtered 330. For example, the stability of the buffy coat layer over time may be monitored. Statistically bad data points may be excluded. For example, if it appears that the buffy coat layer is not stable, the platelet interface may be used as the nominal interface with the red cell layer. The stability of the red blood cell interface may be checked using the standard deviation of a moving 500 ms window of the red cell interface position data. Otherwise the data may be checked against predetermined limits, and excluded as invalid if outside those limits. The results of the data acquisition are reported 332, for example, by reporting measured cell flux values and average interface locations. The data for the processed image of the interfaces can then be logged 334.

The collect port intensity tool 274 measures cell flux through the collect port as a function of light intensity. This subroutine adds 336 new collect port intensity data to a collect port data array. The data should only be added if the collect port intensity has been measured successfully. Relevant data comprises the detected light intensity (reflected or transmitted) in the collect port monitoring region 204 and the flow rate as controlled by the collect port pump. From this data, a cell flux through the collect port can be computed 338.

Post-process 340 re-sets certain measurement conditions such as the lighting or strobe conditions. Post exit 342 signals the completion of a measure mode subroutine.

A complete measure mode subroutine 344 follows the same programming structure as described above, with a pre-enter 346 segment invoking the subroutine, a pre-process 348 setting initial parameters, a process of resetting the lighting 350, a post process 352 normalizing parameters and a pre-exit 354 reporting the completion of the subroutine.

This general operation provides for machine control of the aphaeresis machine by identifying the interface location in real time and adjusting pump and rotor speeds to control the interface location accurately and consistently while determining cell flux through the collect port to monitor production of the selected blood product. More detailed descriptions of certain subroutines used in the program described above will now be given.

The interface measurement tool 272 provides the capability of detecting interface positions without regard to lighting or blood composition changes. The interface measurement tool 272 combines a series of mathematical algorithms to process data acquired from on the order of 790,000 pixels, each pixel having an intensity value between 0 and 255. Using a Synchronization and Timing Controller (STC), the camera captures an image or "frame" including the phase boundary monitoring region 202. Within the frame, the software starts from a defined (X,Y) coordinate pair and moves regularly through the image, calculating the change in intensity of the pixels. For example, the calculation may commence in the upper left region and move horizontally across the image, then move down a row and again sweep across the image until the entire image has been processed. The data processing preferably comprises one or more of six distinct steps or processes. The effect of these processes is illustrated in Fig. 6 and has been described generally above.

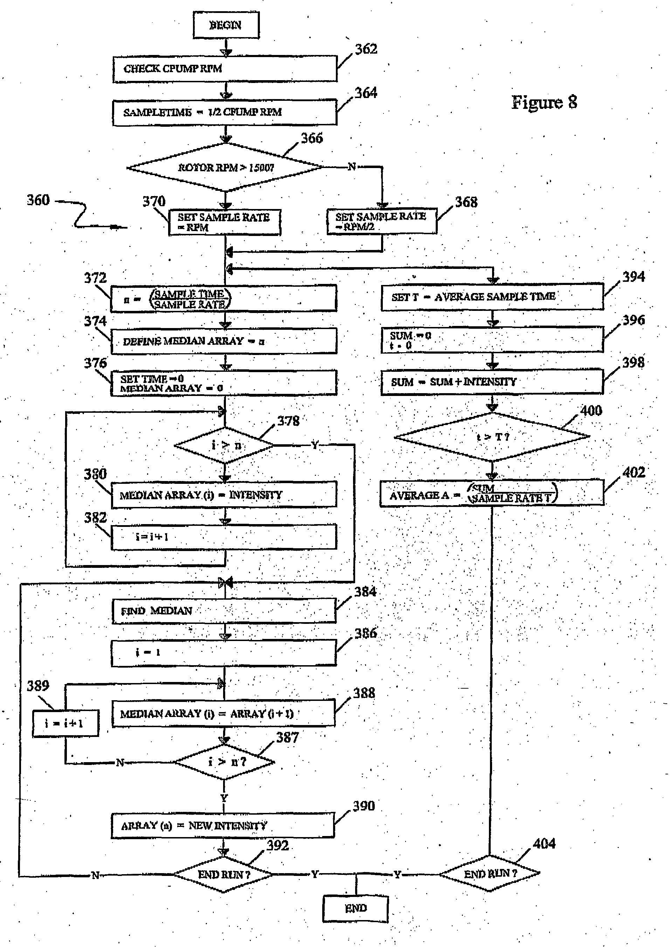

Figure 8 illustrates the acquisition 360 of two measures of the collect port intensity, the arithmetic average A and the median B. Preferably, the average A will be calculated over a relatively short period of time, for example, 0.5 seconds, while the median B will be selected over a longer period, for example 20 to 30 seconds. The period of selection for the median, however, is a rolling period, that is, the oldest data point is discarded for each new datum added to the data set. Therefore, the median B is refreshed at the sampling rate, but is relatively stable because of the size of the data set from which the median is selected. To locate the median B, a set of data is collected based on the collect pump speed. The collect pump speed is determined 362, and a sample time is set 364 as a fraction (e.g., 1/2) or multiple of the pump speed. A sample rate is selected based on the rotor speed. For example, if the rotor speed is greater 366 than 1500 RPM, the sample rate may be set 368 at 1/2 the rotor speed. Otherwise the sample rate could be set 370 at the rotor speed. The adaptation of the sample rate to the rotor speed is limited primarily by the refresh capability of the camera and by the data processing speed of the microprocessor implementing these detection algorithms. A number n of samples will be taken 372 and a median data array is defined 374 for the data. Variables "set time" and the median data array are initialized 376. The index i is tested 378 and incremented 382 until each element of the median array is populated 380 with an intensity measurement from the sampling region of the outflow tube. This measurement is preferably the average of the pixels focused on the selected sampling region. Once the n samples have been taken, the median of the samples is located 384, that is, the sample value that has half of the samples above the median value and half the samples below the median value. The index i is again set 386 to 1 and the samples in the median array 388 are sifted by testing 387 and incrementing 389 the index i, eliminating the oldest value. A new intensity sample is added 390 to the median array. A new median value B is found 384 for each new cycle until the end of the run 392, that is, until the blood processing is complete.

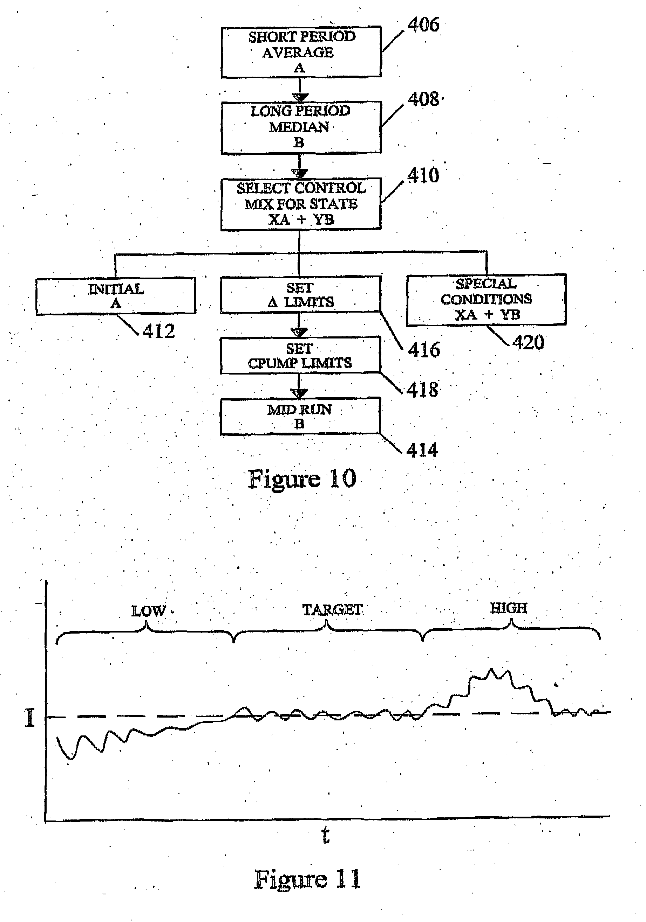

In parallel, an average value A of the intensity is calculated. A sample time T is selected 394. A variable SUM and a time t are initialized 396. Intensity samples are added 398 to the SUM until t reaches 400 the sample time T. The arithmetic average A is calculated 402 as SUM divided by the product of the sample rate and the sample time T. The average intensity A is calculated for a new period T until the end of the run is detected 404. As illustrated in Figure 10, control of fluid flow using the intensity of detected light from the outflow tube comprises developing 406 the average A over a short term and developing 408 the median B over a longer term. A combination control may also be calculated 410 by computing a weighted combination of the average A and the mean B. Fractional coefficients X and Y may be selected based on general characteristics of expected donors or on experience with a particular donors. Values of X and Y equal to 0.5 would give equal weight to A and B. A value of 0.8 for X and 0.2 for Y would bias control toward the average A, a generally faster response. A value of 0.2 for X and 0.8 for Y would favor a slower, more stable response to changes in the composition of the fluid in the outflow tube. Each of these factors may be selected as the current control parameter based on detected operating conditions. For example, the average A may be used during initial conditions 412, when flow is being first established. As shown in Figure 11, this may be a period when the intensity is low, and it is desired to approach the target intensity (shown by a dashed line) smoothly so that there would be limited overshoot. Higher intensity might indicate the presence of red blood cells, and it would be undesirable to allow a large quantity of red blood cells to enter the outflow tube if white blood cells or plasma were being collected. When a relatively steady state has been achieved, the apparatus may shift 414 to control based on the median B. In this condition, it may also be advisable to set maximum and minimum limits on the control pump speed 418 and the permitted change Δ in pump speed 416, so that in the target area, rapid changes in flow conditions would be limited. Under special conditions, a combination control XA + YB may be selected 420. For example If the intensity becomes high, as shown in Figure H, indicating the presence of unwanted red blood cells, a rapid return to the target intensity may be required, even if there would be some overshoot into a low intensity condition.

It will be apparent to those skilled in the art that various modifications and variations can be made to the structure and methodology of the present invention without departing from the scope of the invention. Rather, the invention is intended to cover modifications and variations provided they come within the scope of the following claims.

## Claims

1. A centrifuge blood processing system for separating fluid components, said system comprising:
a separation chamber (28) arranged to rotate about a central rotation axis, said separation chamber having an outflow passage (112),
at least one pump (158, 160, 162) regulating fluid flow in said separation chamber;
a light source (42) in optical communication with said centrifuge blood processing system, said light source (42) providing an incident light beam (54) for illuminating an observation region (202) on said centrifuge blood processing system and a viewing region (204) on said outflow passage (112);
a first detector (46) in optical communication with said separation chamber to receive and detect said light from said observation region (202);
a second detector (46) in optical communication with said outflow passage (112);
a computational apparatus distinguishing one or more phase boundaries in said observation region (202) and further distinguishing fluid composition in said viewing region (204) as a function of light intensity received from said viewing region (204);
a controller (164) regulating speed of said at least one pump (158, 160, 162) or of said separation chamber (28) in response to signals from said computational apparatus;
**characterised in that** said system is arranged to calculate:
(a) a short-period arithmetic average A of intensity in said outflow passage;
(b) a longer-period median average B of intensity in said outflow passage; and
(c) a weighted combination of arithmetic average A and median average B;
wherein said system is arranged to bias control in favour of said median average B when a relatively steady state has been achieved.

2. The centrifuge blood processing system of claim 1, further comprising a camera (72) having a two-dimensional field of view, and wherein said first detector comprises a first discrete area in said field of view and said second detector comprises a second discrete area in said field of view.

3. The centrifuge blood processing system of claim 2, wherein said camera (72) comprises a two dimensional array of pixels.

4. The centrifuge blood processing device of claim 1, further comprising means for selectively controlling said controller in response to either said distinguished phase boundaries in said observation region (202) or said fluid composition in said outflow passage (112).

5. The centrifuge blood processing device of claim 4, wherein said computational apparatus is arranged to select control based on said phase boundaries during transient changes in fluid conditions in said separation chamber (28).

6. The centrifuge blood processing device of claim 4, wherein said computational apparatus is arranged to select control based on said fluid composition in said outflow passage (112) during steady-state flow conditions in said separation chamber (28).

7. The centrifuge blood processing system of claim 1, wherein said computational apparatus distinguishes an average light intensity in said viewing region (204) of said outflow passage (112) over a pre-selected time, and said controller regulates said speed based on said average light intensity.

8. The centrifuge blood processing system of claim 7, wherein said average light intensity is a median value selected from a set of discrete light intensity measurements.

9. The centrifuge blood processing system of claim 8, wherein said set of discrete light intensity measurements is a rolling set.

10. The centrifuge blood processing system of claim 9, wherein said rolling set is formed by deleting the oldest light intensity measurement from the set and by adding a new light intensity measurement to the set.

11. The centrifuge blood processing system of claim 1, wherein said first detector uses image processing procedures comprising scanning a field of pixel values to detect a plurality of linked adjacent edges on phase boundaries between blood components and wherein said linked adjacent edges are recognized as a phase boundary if the length of said linked adjacent edges is greater than a predetermined minimum length.

12. The centrifuge blood processing system of claim 11, wherein said minimum length is at least 75% of a width of said observation region.

13. The centrifuge blood processing system of claim 1, wherein said first detector uses image processing procedures comprising scanning a field of pixel values to detect a phase boundary between blood components; and assign pixels to regions with respect to said detected boundaries.

14. The centrifuge blood processing system of claim 13, wherein said computational apparatus determines an average intensity for pixels in each of said regions.

15. The centrifuge blood processing system of claim 2, wherein said two-dimensional detector samples fewer pixels in a direction parallel to an expected phase boundary between said fluid components as compared to a number of pixels sampled in a direction perpendicular to said expected phase boundary.

16. The centrifuge blood processing system of claim 1, wherein said first detector uses image processing procedures comprising receiving intensities for selected pixels and smoothing oscillations in phase boundaries between blood components.

17. The centrifuge blood processing system of claim 1, wherein said first detector uses image processing procedures comprising determining a set of gradients of said pixel values.

18. A method for separating fluid components, said method comprising:
providing a separation chamber (28) rotating about a central rotation axis, said separation chamber having an outflow passage (112),
providing at least one pump (158, 160, 162) regulating fluid flow in said separation chamber (28);
providing an incident light beam (54) for illuminating an observation region (202) on said centrifuge blood processing system and a viewing region (204) on said outflow passage;
receiving and detecting said light from said observation region (202) at a first detector in optical communication with said separation chamber;
detecting said light from said viewing region (204) at a second detector in optical communication with said outflow passage;
distinguishing one or more phase boundaries in said observation region (202);
distinguishing fluid composition in said viewing region (204) as a function of light intensity received from said viewing region;
regulating the speed of said at least one pump (158, 160, 162) or of said separation chamber (28) in response to said distinguishing steps;
calculating:
(a) a short-period arithmetic average A of intensity in said outflow passage;
(b) a longer-period median average B of intensity in said outflow passage; and
(c) a weighted combination of arithmetic average A and median average B;
shifting control in favour of said median average B when a relatively steady state has been achieved.

## Patentansprüche

1. Zentrifugenblutverarbeitungssystem zum Trennen von Fluidkomponenten, wobei das System Folgendes umfasst:
eine Trennkammer (28), die dafür ausgelegt ist, sich um eine zentrale Drehachse zu drehen, wobei die Trennkammer einen Auslassdurchgang (112) aufweist,
mindestens eine Pumpe (158, 160, 162), die den Fluidstrom in der Trennkammer regelt;
eine Lichtquelle (42) in optischer Verbindung mit dem Zentrifugenblutverarbeitungssystem, wobei die Lichtquelle (42) einen einfallenden Lichtstrahl (54) zum Beleuchten eines Beobachtungsbereichs (202) an dem Zentrifugenblutverarbeitungssystem und eines Sichtbereichs (204) an dem Auslassdurchgang (112) bereitstellt;
einen ersten Detektor (46) in optischer Verbindung mit der Trennkammer, um das Licht von dem Beobachtungsbereich (202) zu empfangen und zu erfassen;
einen zweiten Detektor (46) in optischer Verbindung mit dem Auslassdurchgang (112);
eine Rechenvorrichtung zur Unterscheidung einer oder mehrerer Phasengrenzen in dem Beobachtungsbereich (202) und ferner zur Unterscheidung der Fluidzusammensetzung in dem Sichtbereich (204) als eine Funktion der Lichtintensität, die von dem Sichtbereich (204) empfangen wird;
eine Steuereinheit (164) zur Regelung der Geschwindigkeit der mindestens einen Pumpe (158, 160, 162) oder der Trennkammer (28) als Reaktion auf Signale von der Rechenvorrichtung;
**dadurch gekennzeichnet, dass** das System dafür ausgelegt ist, Folgendes zu berechnen:
(a) einen kurzperiodischen arithmetischen Mittelwert A der Intensität in dem Auslassdurchgang;
(b) einen Medianmittelwert B mit längerer Periode der Intensität in dem Auslassdurchgang; und
(c) eine gewichtete Kombination aus dem arithmetischen Mittelwert A und dem Medianmittelwert B;
wobei das System dafür ausgelegt ist, die Steuerung zugunsten des Medianmittelwerts B vorzuspannen, wenn ein relativ stabiler Zustand erreicht worden ist.

2. Zentrifugenblutverarbeitungssystem nach Anspruch 1, das ferner eine Kamera (72) umfasst, die ein zweidimensionales Sichtfeld aufweist, und wobei der erste Detektor eine erste diskrete Zone in dem Sichtfeld umfasst und der zweite Detektor eine zweite diskrete Zone in dem Sichtfeld umfasst.

3. Zentrifugenblutverarbeitungssystem nach Anspruch 2, wobei die Kamera (72) eine zweidimensionale Anordnung von Pixeln umfasst.

4. Zentrifugenblutverarbeitungsvorrichtung nach Anspruch 1, die ferner Mittel zum selektiven Steuern der Steuereinheit als Reaktion entweder auf die charakteristischen Phasengrenzen in dem Beobachtungsbereich (202) oder die Fluidzusammensetzung in dem Auslassdurchgang (112) umfasst.

5. Zentrifugenblutverarbeitungsvorrichtung nach Anspruch 4, wobei die Rechenvorrichtung dafür ausgelegt ist, die Steuerung basierend auf den Phasengrenzen während transienter Änderungen der Fluidbedingungen in der Trennkammer (28) auszuwählen.

6. Zentrifugenblutverarbeitungsvorrichtung nach Anspruch 4, wobei die Rechenvorrichtung dafür ausgelegt ist, die Steuerung basierend auf der Fluidzusammensetzung in dem Auslassdurchgang (112) während stabiler Strömungsbedingungen in der Trennkammer (28) auszuwählen.

7. Zentrifugenblutverarbeitungssystem nach Anspruch 1, wobei die Rechenvorrichtung eine durchschnittliche Lichtintensität in dem Sichtbereich (204) des Auslassdurchgangs (112) über eine vorgewählte Zeit unterscheidet und die Steuereinheit die Geschwindigkeit basierend auf der durchschnittlichen Lichtintensität regelt.

8. Zentrifugenblutverarbeitungssystem nach Anspruch 7, wobei die durchschnittliche Lichtintensität ein Medianwert ist, der aus einem Satz von diskreten Lichtintensitätsmessungen ausgewählt wird.

9. Zentrifugenblutverarbeitungssystem nach Anspruch 8, wobei der Satz von diskreten Lichtintensitätsmessungen ein Rollsatz ist.

10. Zentrifugenblutverarbeitungssystem nach Anspruch 9, wobei der Rollsatz durch Löschen der ältesten Lichtintensitätsmessung aus dem Satz und durch Hinzufügen einer neuen Lichtintensitätsmessung zu dem Satz gebildet wird.

11. Zentrifugenblutverarbeitungssystem nach Anspruch 1, wobei der erste Detektor Bildverarbeitungsverfahren verwendet, die das Abtasten eines Feldes von Pixelwerten umfassen, um eine Vielzahl von verknüpften benachbarten Kanten an Phasengrenzen zwischen Blutkomponenten zu erfassen, und wobei die verknüpften benachbarten Kanten als Phasengrenze erkannt werden, wenn die Länge der verknüpften benachbarten Kanten größer als eine vorbestimmte Mindestlänge ist.

12. Zentrifugenblutverarbeitungssystem nach Anspruch 11, wobei die Mindestlänge mindestens 75 % einer Breite des Beobachtungsbereichs beträgt.

13. Zentrifugenblutverarbeitungssystem nach Anspruch 1, wobei der erste Detektor Bildverarbeitungsverfahren verwendet, die Folgendes umfassen: Abtasten eines Feldes von Pixelwerten, um eine Phasengrenze zwischen Blutkomponenten zu erfassen; und Zuweisen von Pixeln zu Bereichen in Bezug auf die erfassten Grenzen.

14. Zentrifugenblutverarbeitungssystem nach Anspruch 13, wobei die Rechenvorrichtung eine mittlere Intensität für Pixel in jedem der Bereiche bestimmt.

15. Zentrifugenblutverarbeitungssystem nach Anspruch 2, wobei der zweidimensionale Detektor weniger Pixel in einer Richtung parallel zu einer erwarteten Phasengrenze zwischen den Fluidkomponenten verglichen mit einer Anzahl von Pixeln in einer Richtung senkrecht zur erwarteten Phasengrenze abtastet.

16. Zentrifugenblutverarbeitungssystem nach Anspruch 1, wobei der erste Detektor Bildverarbeitungsverfahren verwendet, die das Empfangen von Intensitäten für ausgewählte Pixel und das Glätten von Schwingungen in Phasengrenzen zwischen Blutkomponenten umfasst.

17. Zentrifugenblutverarbeitungssystem nach Anspruch 1, wobei der erste Detektor Bildverarbeitungsverfahren verwendet, die das Bestimmen eines Satzes von Gradienten der Pixelwerte umfassen.

18. Verfahren zum Trennen von Fluidkomponenten, wobei das Verfahren Folgendes umfasst:
Bereitstellen einer Trennkammer (28), die sich um eine zentrale Drehachse dreht, wobei die Trennkammer einen Auslassdurchgang (112) aufweist,
Bereitstellen mindestens einer Pumpe (158, 160, 162), die den Fluidstrom in der Trennkammer (28) regelt;
Bereitstellen eines einfallenden Lichtstrahls (54) zum Beleuchten eines Beobachtungsbereichs (202) an dem Zentrifugenblutverarbeitungssystem und eines Sichtbereichs (204) an dem Auslassdurchgang;
Empfangen und Erfassen des Lichts von dem Beobachtungsbereich (202) an einem ersten Detektor, der mit der Trennkammer in optischer Verbindung steht;
Erfassen des Lichts von dem Beobachtungsbereich (204) an einem zweiten Detektor, der mit dem Abflussdurchlass in optischer Verbindung steht;
Unterscheiden einer oder mehrerer Phasengrenzen in dem Beobachtungsbereich (202);
Unterscheiden der Fluidzusammensetzung in dem Sichtbereich (204) als eine Funktion der Lichtintensität, die von dem Beobachtungsbereich empfangen wird;
Regeln der Geschwindigkeit der mindestens einen Pumpe (158, 160, 162) oder der Trennkammer (28) als Reaktion auf die Unterscheidungsschritte;
Berechnen:
(a) eines kurzperiodischen arithmetischen Mittelwerts A der Intensität in dem Auslassdurchgang;
(b) eines Medianmittelwerts B mit längerer Periode der Intensität in dem Auslassdurchgang; und
(c) einer gewichteten Kombination aus dem arithmetischen Mittelwert A und dem Medianmittelwert B;
Umschalten der Steuerung zugunsten des Medianmittelwerts B, wenn ein relativ stabiler Zustand erreicht worden ist.

## Revendications

1. Système de traitement centrifuge du sang permettant de séparer des composants fluidiques, ledit système comprenant :
une chambre de séparation (28) agencée pour tourner autour d'un axe de rotation central, ladite chambre de séparation ayant un passage d'écoulement sortant (112),
au moins une pompe (158, 160, 162) régulant l'écoulement fluidique dans ladite chambre de séparation ;
une source de lumière (42) en communication optique avec ledit système de traitement centrifuge du sang, ladite source de lumière (42) fournissant un faisceau de lumière incident (54) destiné à illuminer une région d'observation (202) sur ledit système de traitement centrifuge du sang et une région de visualisation (204) sur ledit passage d'écoulement sortant (112) ;
un premier détecteur (46) en communication optique avec ladite chambre de séparation pour recevoir et détecter ladite lumière provenant de ladite région d'observation (202) ;
un second détecteur (46) en communication optique avec ledit passage d'écoulement sortant (112) ;
un appareil informatique distinguant une ou plusieurs limites de phase dans ladite région d'observation (202) et distinguant en outre une composition fluidique dans ladite région de visualisation (204) en fonction de l'intensité de lumière reçue de ladite région de visualisation (204) ;
un dispositif de commande (164) régulant la vitesse de ladite au moins une pompe (158, 160, 162) ou de ladite chambre de séparation (28) en réponse à des signaux dudit appareil informatique ;
**caractérisé en ce que** ledit système est agencé pour calculer :
(a) une moyenne arithmétique de période courte A d'intensité dans ledit passage d'écoulement sortant ;
(b) une moyenne médiane de période plus longue B d'intensité dans ledit passage d'écoulement sortant ; et
(c) une combinaison pondérée de la moyenne arithmétique A et de la moyenne médiane B ;
ledit système étant agencé pour inciter la commande en faveur de ladite moyenne médiane B lorsqu'un état relativement stable a été atteint.

2. Système de traitement centrifuge du sang de la revendication 1, comprenant en outre une caméra (72) ayant un champ de vision bidimensionnel, et dans lequel ledit premier détecteur comprend une première zone discrète dans ledit champ de vision et ledit second détecteur comprend une seconde zone discrète dans ledit champ de vision.

3. Système de traitement centrifuge du sang de la revendication 2, dans lequel ladite caméra (72) comprend une matrice bidimensionnelle de pixels.

4. Dispositif de traitement centrifuge du sang de la revendication 1, comprenant en outre un moyen permettant de commander sélectivement ledit dispositif de commande en réponse soit auxdites limites de phase distinguées dans ladite région d'observation (202), soit à ladite composition fluidique dans ledit passage d'écoulement sortant (112).

5. Dispositif de traitement centrifuge du sang de la revendication 4, dans lequel ledit appareil informatique est agencé pour sélectionner une commande sur la base desdites limites de phase durant des changements transitoires de conditions fluidiques dans ladite chambre de séparation (28).

6. Dispositif de traitement centrifuge du sang de la revendication 4, dans lequel ledit appareil informatique est agencé pour sélectionner une commande sur la base de ladite composition fluidique dans ledit passage d'écoulement sortant (112) durant des conditions d'écoulement stable dans ladite chambre de séparation (28).

7. Système de traitement centrifuge du sang de la revendication 1, dans lequel ledit appareil informatique distingue une intensité de lumière moyenne dans ladite région de visualisation (204) dudit passage d'écoulement sortant (112) sur une durée sélectionnée au préalable, et ledit dispositif de commande régule ladite vitesse sur la base de ladite intensité de lumière moyenne.

8. Système de traitement centrifuge du sang de la revendication 7, dans lequel ladite intensité de lumière moyenne est une valeur médiane sélectionnée dans un ensemble de mesures discrètes d'intensité de lumière.

9. Système de traitement centrifuge du sang de la revendication 8, dans lequel ledit ensemble de mesures discrètes d'intensité de lumière est un ensemble en roulement.

10. Système de traitement centrifuge du sang de la revendication 9, dans lequel ledit ensemble en roulement est formé par la suppression de la mesure d'intensité de lumière la plus ancienne de l'ensemble et l'ajout d'une nouvelle mesure d'intensité de lumière à l'ensemble.

11. Système de traitement centrifuge du sang de la revendication 1, dans lequel ledit premier détecteur utilise des procédures de traitement d'image comprenant le balayage d'un champ de valeurs de pixel pour détecter une pluralité de bords adjacents reliés sur des limites de phase entre des composants du sang, et dans lequel lesdits bords adjacents reliés sont reconnus comme limite de phase si la longueur desdits bords adjacents reliés est supérieure à une longueur minimale prédéterminée.

12. Système de traitement centrifuge du sang de la revendication 11, dans lequel ladite longueur minimale vaut au moins 75 % d'une largeur de ladite région d'observation.

13. Système de traitement centrifuge du sang de la revendication 1, dans lequel ledit premier détecteur utilise des procédures de traitement d'image comprenant le balayage d'un champ de valeurs de pixel pour détecter une limite de phase entre des composants du sang ; et attribuer des pixels à des régions vis-à-vis desdites limites détectées.

14. Système de traitement centrifuge du sang de la revendication 13, dans lequel ledit appareil informatique détermine une intensité moyenne pour les pixels dans chacune desdites régions.

15. Système de traitement centrifuge du sang de la revendication 2, dans lequel ledit détecteur bidimensionnel échantillonne un nombre moins élevé de pixels dans une direction parallèle à une limite de phase prévue entre lesdits composants fluidiques en comparaison avec le nombre de pixels échantillonnés dans une direction perpendiculaire à ladite limite de phase prévue.

16. Système de traitement centrifuge du sang de la revendication 1, dans lequel ledit premier détecteur utilise des procédures de traitement d'image comprenant la réception d'intensités pour des pixels sélectionnés et le lissage des oscillations dans des limites de phase entre des composants du sang.

17. Système de traitement centrifuge du sang de la revendication 1, dans lequel ledit premier détecteur utilise des procédures de traitement d'image comprenant la détermination d'un ensemble de gradients desdites valeurs de pixel.

18. Procédé de séparation des composants fluidiques, ledit procédé comprenant :
la fourniture d'une chambre de séparation (28) tournant autour d'un axe de rotation central, ladite chambre de séparation ayant un passage d'écoulement sortant (112),
la fourniture d'au moins une pompe (158, 160, 162) régulant l'écoulement fluidique dans ladite chambre de séparation (28) ;
la fourniture d'un faisceau de lumière incident (54) destiné à illuminer une région d'observation (202) sur ledit système de traitement centrifuge du sang et une région de visualisation (204) sur ledit passage d'écoulement sortant ;
la réception et la détection de ladite lumière provenant de ladite région d'observation (202) au niveau d'un premier détecteur en communication optique avec ladite chambre de séparation ;
la détection de ladite lumière provenant de ladite région de visualisation (204) au niveau d'un second détecteur en communication optique avec ledit passage d'écoulement sortant ;
la distinction d'une ou plusieurs limites de phase dans ladite région d'observation (202) ;
la distinction d'une composition fluidique dans ladite région de visualisation (204) en fonction de l'intensité de lumière reçue de ladite région de visualisation ;
la régulation de la vitesse de ladite au moins une pompe (158, 160, 162) ou de ladite chambre de séparation (28) en réponse auxdites étapes de distinction ;
le calcul :
(a) d'une moyenne arithmétique de période courte A d'intensité dans ledit passage d'écoulement sortant ;
(b) d'une moyenne médiane de période plus longue B d'intensité dans ledit passage d'écoulement sortant ; et
(c) d'une combinaison pondérée de la moyenne arithmétique A et de la moyenne médiane B ;
le changement de commande en faveur de ladite moyenne médiane B lorsqu'un état relativement stable a été atteint.
